# FASCICULE DE BREVET EUROPEEN

(11) **EP 0 552 106 B1**
(45) Date de publication et mention de la délivrance du brevet: **07.02.1996**
(21) Numéro de dépôt: 93400086.0
(22) Date de dépôt: 15.01.1993
(51) Int. Cl.: C07K 7/18, A61K 38/04

(54) **Peptides ayant une activité antagoniste pour la bradykinine**
Peptide mit Bradykinin-antagonistischer Wirkung
Peptide antagonists of bradykinin

(30) Priorité: 17.01.1992 FR 9200438
(43) Date de publication de la demande: 21.07.1993
(73) Titulaire: ADIR ET COMPAGNIE, F-92415 Courbevoie Cédex (FR)
(72) Inventeur: Fauchere, Jean-Luc, F-92210 Saint-Cloud (FR); Thurieau, Christophe, F-92100 Boulogne sur Seine (FR); Canet, Emmanuel, F-75005 Paris (FR)

(56) Documents cités:
- FR-A- 2 111 897
- EDITEURS: SMITH ET AL 'Peptides - Proceedings of the 12th American Peptide Symposium, Juin 1991, Cambridge, USA' 1992 , ESCOM , LEIDEN, HOLLANDE

## Description

La présente invention concerne de nouveaux dérivés pseudopeptidiques à activité antagoniste de la bradykinine, leur procédé de synthèse et les compositions pharmaceutiques qui les contiennent.

La bradykinine, nonapeptide naturel, est connue comme médiateur des réactions inflammatoires et douloureuses ainsi que dans les états d'hypotension.

La bradykinine a de plus des effets contracturants sur le muscle lisse en particulier trachéal, utérin ou intestinal. En clinique, la bradykinine a été impliquée dans la physiopathologie des états de choc, des réactions inflammatoires, de l'asthme et de l'hyperréactivité bronchique, des rhinites allergiques ou virales, de la pancréatite, des arthrites, du "dumping" syndrome post-gastrectomie, du psoriasis, de l'oedème angioneurotique héréditaire, de la migraine.

La demande EP 0370453 décrit des antagonistes de la bradykinine à structure pseudopeptidique ayant pour formule :
A-B-C-E-F-K-(D)Tic-G-M-F-I
et parmi ceux-ci le décapeptide de formule :
H-(D)Arg-Arg-Pro-Hyp-Gly-Thia-Ser-(D)Tic-Oic-Arg-OH
où Arg représente le résidu arginyle,
Pro représente le résidu prolyle,
Hyp représente le résidu 4-hydroxyprolyle,
Gly représente le résidu glycyle,
Thia représente le résidu β-(2-thiényl)alanyle,
Ser représente le résidu séryle,
Tic représente le résidu tétrahydroisoquinoléine-3-carbonyle,
de formule : 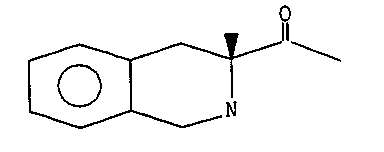
Oic le résidu octahydroindole-2-carbonyle de formule : 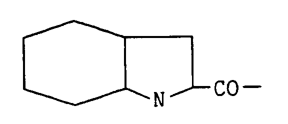
le sigle (D) précédant le symbole d'un des acides aminés mentionnés ci-dessus signifiant que cet acide aminé a la configuration (D). En l'absence du sigle (D), les acides aminés sont réputés avoir la configuration (L).

La présente invention décrit plus spécifiquement le nonapeptide de formule :
G-Arg-Pro-Hyp-Gly-Thia-Ser-Tic-Oic-Arg-OH
où G représente le radical 4-guanidinobenzoyle de formule : 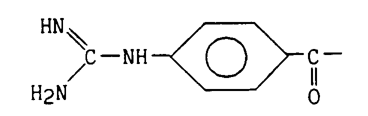
et Arg représente le résidu arginyle,
Pro représente le résidu prolyle,
Hyp représente le résidu 4-hydroxyprolyle,
Gly représente le résidu glycyle,
Thia représente le résidu β-(2-thiényl)alanyle,
Ser représente le résidu séryle,
Tic le résidu 1,2,3,4-tétrahydroisoquinoléine-3-carbonyle,
Oic représente le résidu octahydroindole-2-carbonyle,
chaque amino-acide de la séquence peptidique ayant au niveau de son carbone α la configuration D ou L, ses énantiomères, diastéréoisomères et épimères ainsi que ses sels d'addition à un acide ou à une base pharmaceutiquement acceptable.

Le composé préféré de la présente invention est le composé de formule :
G-Arg-Pro-Hyp-Gly-Thia-Ser-(D)Tic-Oic-Arg-OH
dans laquelle :
(D)Tic possède la configuration D au niveau de son carbone α, les autres acides aminés de la séquence peptidique possèdent au niveau de leur carbone α la configuration L, Hyp est de configuration 4R, Oic est de configuration (2S,3aS,7aS),
ainsi que ses sels d'addition à un acide ou à une base pharmaceutiquement acceptable.

Parmi les acides pharmaceutiquement acceptables on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables on peut citer à titre non limitatif l'hydroxyde de sodium, l'hydroxyde de potassium, la triéthylamine, la tertbutylamine, etc...

Ce composé, outre le fait qu'il soit nouveau, est doué de propriétés plus intéressantes que celles du dérivé préféré de la demande EP 0370453.

La structure originale nonapeptidique du produit de l'invention, en particulier l'absence de fonction Nα-amino terminale, lui assure in vivo une plus grande résistance aux aminopeptidases que la structure décapeptidique du composé préféré de la demande EP 0370453.

D'autre part, sa structure de nonapeptide lui confère 9 centres asymétriques, soit un centre asymétrique de moins que dans le cas du décapeptique décrit dans la demande EP 0370453, ce qui représente un avantage non négligeable lors d'une synthèse industrielle.

De plus, dans les tests in vitro réalisés afin de rechercher l'activité intrinsèque, le dérivé de l'invention s'est révélé significativement meilleur que le produit préféré de la demande EP 0370453.

L'invention s'étend aussi au procédé de préparation des dérivés de l'invention qui peuvent être obtenus par différentes méthodes telles que la synthèse séquentielle sur phase solide, la synthèse de fragments et leur couplage en solution, la synthèse enzymatique, la synthèse génétique par clonage et expression de gènes dans des bactéries transformées ou par des combinaisons diverses de ces techniques.

Les méthodes générales de la synthèse des peptides sur phase solide ont été décrites par B.W. ERICKSON et R.B. MERRIFIELD ("The Proteins", Solid-Phase Peptide Synthesis, 3^{ème} édition, volume II, 257-527, 1976).

La synthèse sur phase solide peut se réaliser sur un automate qui effectue de façon répétitive et programmable des cycles de déprotection, couplage et lavages nécessaires à l'introduction séquentielle des acides aminés dans la chaîne peptidique. L'acide aminé, de préférence C-terminal est fixé sur une résine conventionnellement utilisée pour la préparation de polypeptides, de préférence un polystyrène réticulé à l'aide de 0,5 à 3,0 % de divinylbenzène et muni de restes activés tels que le chlorométhylène ou l'hydroxyméthylène qui permettent la fixation covalente du premier acide aminé sur la résine. Le choix approprié de la résine permet la fixation d'une fonction C-terminale acide carboxylique, amide ou alcool. Le choix du site de couplage des fragments est souvent déterminé de façon à minimiser les risques de racémisation. Trois sites de couplage sont, par exemple, la fonction C-terminale de la proline (en position 2), de l'hydroxyproline (en position 3) et de la glycine (en position 4).

Les acides aminés sont alors introduits un à un dans l'ordre déterminé par l'opérateur. Chaque cycle de synthèse correspondant à l'introduction d'un acide aminé, comporte une déprotection, de préférence N-terminale de la chaîne peptidique, des lavages successifs destinés à éliminer les réactifs ou à gonfler la résine, un couplage avec activation de l'acide aminé et de nouveaux lavages. Chacune de ces opérations est suivie d'une filtration réalisée grâce à la présence d'un verre fritté incorporé au réacteur dans lequel s'effectue la synthèse.

Les réactifs de couplage utilisés sont des réactifs classiques de la synthèse peptidique comme le dicyclohexylcarbodiimide (DCC) et l'hydroxybenzotriazole (HOBT) ou le benzotriazol-1-yl-oxytris (diméthylamino) phosphonium hexafluorophosphate (BOP) ou encore le diphenylphosphorylazide (DPPA).

Des activations par formation d'anhydrides mixtes sont également possibles.

Chaque acide aminé est introduit dans le réacteur en excès (quadruple par exemple), par rapport au degré de substitution de la résine et en quantité environ équivalente par rapport aux agents de couplage. La réaction de couplage peut être vérifiée à chaque étape de la synthèse par le test de réaction à la ninhydrine décrit par E. KAISER et Coll. (Analyt. Biochem., 34, 595, 1970).

Après assemblage de la chaîne peptidique sur la résine, un traitement par un acide fort tel que l'acide trifluoroacétique, ou l'acide fluorhydrique en présence d'anisole, d'éthanedithiol ou de 2-méthylindole sert à séparer le peptide de la résine ainsi qu'à libérer éventuellement le peptide de ses groupes protecteurs. Le composé est alors purifié par des techniques classiques de purification, notamment chromatographiques.

Les peptides de la présente invention peuvent être également obtenus par couplage en solution de fragments peptidiques sélectivement protégés, qui peuvent être préparés soit sur phase solide, soit en solution. Les méthodes générales de la synthèse des peptides en solution sont, par exemple, décrits par F.M. FINN et K. HOFMAN (The Proteins, 3^{ème} édition, volume II, p.105-253, 1976). L'emploi des groupes protecteurs et la mise à profit de leur stabilité différentielle est analogue aux méthodes sur phase solide à l'exception de l'attachement de la chaîne peptidique sur la résine. Le groupe carboxylique C-terminal est protégé, par exemple, par un ester méthylique ou une fonction amide. Les méthodes d'activation lors des couplages sont également analogues à celles employées dans la synthèse sur phase solide.

Un procédé spécifique de préparation par synthèse séquentielle sur phase solide du composé de l'invention est caractérisé en ce que l'on fait réagir sur une résine substituée par Fmoc-Arg-OH, les amino-acides successifs pour conduire à l'ensemble H-Pro-Hyp-Gly-Thia-Ser-(D)Tic-Oic-Arg-résine sur lequel ont met finalement en réaction G-Arg(Pmc)-OH.

Les composés de l'invention possèdent des propriétés pharmacologiques très intéressantes, en particulier antagonistes de la bradykinine, dans l'inflammation et la broncoconstriction.

A ce titre, ils peuvent être utilisés avec profit dans un certain nombre d'indications thérapeutiques comme les traumatismes, les écorchures, les brûlures, les éruptions cutanées, eczémas, erythèmes, oedèmes, angines, l'arthrite, l'asthme, les allergies, les rhinites, les chocs anaphylactiques, les inflammations, les hypotensions artérielles, les douleurs, les prurits et les insuffisances de mobilité des spermatozoïdes.

La présente invention a également pour objet les compositions pharmaceutiques renfermant comme principe actif au moins un composé de l'invention ou un de ses sels d'addition à un acide ou à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, crèmes, pommades, gels dermiques, les aérosols, les ampoules buvables et injectables...

La posologie varie selon l'âge et le poids du patient, la nature et la sévérité de l'affection ainsi que la voie d'administration.

Celle-ci peut être orale, nasale, rectale ou parentérale. D'une manière générale, elle s'échelonne entre 5 µg/kg et 5 mg/kg pour un traitement en une ou plusieurs prises par 24 heures.

### EXEMPLE 1 : G-Arg-Pro-Hyp-Gly-Thia-Ser-(D)Tic-Oic-Arg-OH, trifluoroacétate

Le résidu de l'acide octahydroindole-2-carboxylique (Oic) est de configuration (2S,3aS,7aS).

Le composé de l'exemple 1 est synthétisé à partir de 2 g d'une résine substituée par 0,33 mmol/g de Fmoc-Arg-OH et suivant le protocole répétitif suivant :

| N° opération | Fonction | Solvant/Réactif | Répétition/temps |
|---|---|---|---|
| 1 | lavage | DMF | 2 × 2 min |
| 2 | déprotection | 20 % pipéridine/DMF | 1 × 5 min |
| 3 | déprotection | 20 % pipéridine/DMF | 1 × 15 min |
| 4 | lavage | DMF | 3 × 2 min |
| 5 | lavage | dichlorométhane | 3 × 2 min |
| 6 | couplage | acide aminé protégé activé | 1 × 90 min |
| 7 | lavage | DMF | 3 × 2 min |
| 8 | lavage | isopropylique alcool | 3 × 2 min |
| 9 | lavage | dichlorométhane | 3 × 2 min |

Chacune de ces opérations effectuée dans 30 ml de solvant, sous agitation et à température ambiante, est suivie d'une filtration à travers un verre fritté incorporé à la cellule de verre (réacteur) dans laquelle la synthèse progresse. Le filtre retient la résine sur laquelle est fixée la chaîne peptidique croissante.

Les acides aminés protégés choisis ont été introduits dans l'ordre suivant : Fmoc-Oic-OH, Fmoc-(D)Tic-OH, Fmoc-Ser(tBu)-OH, Fmoc-Thia-OH, Fmoc-Gly-OH, Fmoc-Hyp(tBu)-OH, Fmoc-Pro-OH et G-Arg(Pmc)-OH.

L'activation en vue du couplage (opération 6) est obtenue à chaque cycle par dissolution de 4 équivalents (2.64 mmoles) de l'acide aminé protégé avec 360 mg d'HOBt dans 30 ml de DMF, puis après 30 minutes à température ambiante, par addition de 618 mg de DCC. Cette solution est alors immédiatement introduite dans la cellule de réaction avec 10 ml de dichlorométhane.

A la fin des huit cycles correspondant à la fixation séquentielle de huit acides aminés, on a ainsi obtenu avec l'arginine C-terminale, un nonapeptide protégé sur ses chaînes latérales et fixé en position C-terminale sur la résine. La résine est alors traitée par un mélange d'acide trifluoroacétique (18 ml), dichlorométhane (1 ml) et anisol (1 ml) pendant 90 minutes à température ambiante. Le filtrat et les solvants de lavage de la résine (3 × 20 ml dichlorométhane) sont réunis et évaporés à sec. Le produit est suspendu dans l'éther, filtré et séché puis purifié par CLHP préparative, sur colonne C₁₈ (diamètre interne : 47 mm, longueur : 300 mm) et lyophilisé.

L'analyse du produit obtenu est réalisé après décomposition de celui-ci en acides aminés par hydrolyse dans de l'acide chlorhydrique 6N pendant 18 heures à 110°C et dosage quantitatif des acides aminés obtenus par CLHP. Cette analyse est conforme aux normes habituellement exigées.

### EXEMPLE 2 : Etude pharmacologique du dérivé de l'invention : veine jugulaire de lapin : récepteur B₂

La bradykinine engendre une contraction qui est inhibée par le produit de l'exemple 1. Le pA₂ (logarithme négatif de la concentration molaire du dérivé de l'invention qui oblige à doubler la concentration de bradykinine pour obtenir le même effet) est de 9,73 ± 0,1 (n = 5).
Dans les mêmes conditions, le pA₂ du dérivé préféré de la demande EP 0370453 est de 9,23 ± 0,45 soit une différence en faveur des dérivés de l'invention.

### EXEMPLE 3 : Mesure in vivo de l'activité anti-inflammatoire du dérivé de l'invention

L'activité anti-inflammatoire du dérivé de l'invention a été mesurée en utilisant le modèle de l'oedème de la patte de rat induite par injection de carragénine (WINTER G. A. et coll. Proc. Soc. Exp. Biol. Med., 3, 544-547, 1960).
Cette étude est effectuée sur rats mâles de 180-210 g par lot de 8. Les dérivés de l'invention sont administrés par voie I.V. au temps 0 précédant immédiatement l'injection de carragénine par voie sous-cutanée dans la plante de la patte arrière droite du rat (Carragénine lambda type IV, sigma, solution à 1 %, volume injecté 0,1 ml). Le volume de la patte aux différents temps de l'expérimentation est mesurée par plethysmographie. L'inhibition de l'oedème au temps 3 heures est calculée par rapport à un lot contrôle ayant reçu le véhicule et exprimée en pourcentage d'inhibition.
Dans ces conditions, le composé de l'exemple 1 engendre une inhibition de 59 % au temps 3 heures à la dose de 0,1 mg/kg I.V.

### EXEMPLE 4 : Composition pharmaceutique

### Pommade à 5 µg/ml du peptide de l'exemple 1

| | |
|---|---|
| G-Arg-Pro-Hyp-Gly-Thia-Ser(D)Tic-Oic-Arg-OH | 50 mg |
| Polyéthylène glycol Q.S.P. | 100 ml |

### Soluté injectable

| | |
|---|---|
| G-Arg-Pro-Hyp-Gly-Thia-Ser-(D)Tic-Oic-Arg-OH | 0,5 mg |
| Eau distillée pour préparations injectables Q.S.P. | 25 ml |

## Revendications (Revendications pour l'(les) Etat(s) contractant(s) suivant(s): AT, BE, CH, DE, DK, FR, GB, GR, IE, IT, LI, LU, NL, PT, SE)

1. Composé de formule (I) :
G-Arg-Pro-Hyp-Gly-Thia-Ser-Tic-Oic-Arg-OH (I)
où G représente le radical 4-guanidinobenzoyle de formule : 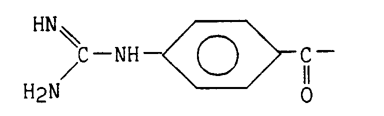 et Arg représente le résidu arginyle,
Pro représente le résidu prolyle,
Hyp représente le résidu 4-hydroxyprolyle,
Gly représente le résidu glycyle,
Thia représente le résidu β-(2-thiényl)alanyle,
Ser représente le résidu séryle,
Tic le résidu 1,2,3,4-tétrahydroisoquinoléine-3-carbonyle,
Oic représente le résidu octahydroindole-2-carbonyle,
chaque amino-acide de la séquence peptidique ayant au niveau de son carbone α la configuration D ou L,
ses énantiomères, diastéréoisomères ainsi que ses sels d'addition à un acide ou à une base pharmaceutiquement acceptable.

2. Composé de formule (I) selon la revendication 1 qui est le composé de formule :
G-Arg-Pro-Hyp-Gly-Thia-Ser-(D)Tic-Oic-Arg-OH
dans laquelle :
(D)Tic représente le radical 1,2,3,4-tétrahydroisoquinoléine-3-carbonyle, de configuration D au niveau de son carbone α, les autres acides aminés de la séquence peptidique possèdent au niveau de leur carbone α la configuration L, Hyp est de configuration 4R, Oic est de configuration (2S,3aS,7aS),
ainsi que ses sels d'addition à un acide ou une base pharmaceutiquement acceptable.

3. Procédé de préparation du composé de formule (I) selon la revendication 1 caractérisé en ce qu'il peut être obtenu par synthèse séquentielle sur phase solide à partir d'amino-acides protégés, par synthèse de fragments peptidiques en solution,
éventuellement par combinaison de ces deux techniques.

4. Procédé de préparation du composé de formule (I) selon l'une quelconque des revendications 1 ou 2 caractérisé en ce qu'il est obtenu par synthèse séquentielle sur phase solide conduisant, à partir d'une résine substituée par Fmoc-Arg-OH, à l'ensemble H-Pro-Hyp-Gly-Thia-Ser-(D)Tic-Oic-Arg-résine que l'on met en réaction directement avec G-Arg(Pmc)-OH conduisant, après déprotection, au composé de formule (I).

5. Compositions pharmaceutiques contenant comme principe actif au moins un composé de formule (I) selon l'une quelconque des revendications 1 ou 2, seul ou en combinaison avec un ou plusieurs excipients ou véhicules pharmaceutiquement acceptables.

6. Compositions pharmaceutiques selon la revendication 5 contenant au moins un principe actif selon l'une quelconque des revendications 1 ou 2 utiles en tant qu'antagoniste de bradykinine dans le traitement des traumatismes d'origine variée, des troubles inflammatoires, de la douleur, des états de chocs, des troubles allergiques, de l'hyperréactivité bronchique et de l'insuffisance de mobilité des spermatozoïdes.

## Revendications (Revendications pour l'(les) Etat(s) contractant(s) suivant(s): ES)

1. Procédé de préparation du composé de formule (I) :
G-Arg-Pro-Hyp-Gly-Thia-Ser-Tic-Oic-Arg-OH (I)
où G représente le radical 4-guanidinobenzoyle de formule : 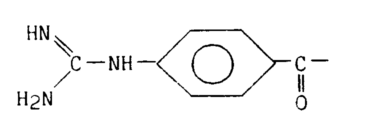 et Arg représente le résidu arginyle,
Pro représente le résidu prolyle,
Hyp représente le résidu 4-hydroxyprolyle,
Gly représente le résidu glycyle,
Thia représente le résidu β-(2-thiényl)alanyle,
Ser représente le résidu séryle,
Tic le résidu 1,2,3,4-tétrahydroisoquinoléine-3-carbonyle,
Oic représente le résidu octahydroindole-2-carbonyle,
chaque amino-acide de la séquence peptidique ayant au niveau de son carbone α la configuration D ou L,
ses énantiomères, diastéréoisomères ainsi que ses sels d'addition à un acide ou à une base pharmaceutiquement acceptable,
caractérisé en ce qu'il peut être obtenu par synthèse séquentielle sur phase solide à partir d'amino-acides protégés, par synthèse de fragments peptidiques en solution,
éventuellement par combinaison de ces deux techniques.

2. Procédé de préparation du composé de formule (I) selon la revendication 1 qui est le composé de formule :
G-Arg-Pro-Hyp-Gly-Thia-Ser-(D)Tic-Oic-Arg-OH
dans laquelle :
(D)Tic représente le radical 1,2,3,4-tétrahydroisoquinoléine-3-carbonyle, de configuration D au niveau de son carbone α, les autres acides aminés de la séquence peptidique possèdent au niveau de leur carbone α la configuration L, Hyp est de configuration 4R, Oic est de configuration (2S,3aS,7aS),
ainsi que ses sels d'addition à un acide ou une base pharmaceutiquement acceptable.

3. Procédé de préparation du composé de formule (I) selon la revendication 1 caractérisé en ce qu'il peut être obtenu par synthèse séquentielle sur phase solide conduisant, à partir d'une résine substituée par Fmoc-Arg-OH, à l'ensemble H-Pro-Hyp-Gly-Thia-Ser-(D)Tic-Oic-Arg-résine que l'on met en réaction directement avec G-Arg(Pmc)-OH conduisant, après déprotection, au composé de formule (I).

## Claims (Claims for the following Contracting State(s): AT, BE, CH, DE, DK, FR, GB, GR, IE, IT, LI, LU, NL, PT, SE)

1. Compound of formula (I):
G-Arg-Pro-Hyp-Gly-Thia-Ser-Tic-Oic-Arg-OH (I)
wherein:
G represents the 4-guanidinobenzoyl radical of the formula: 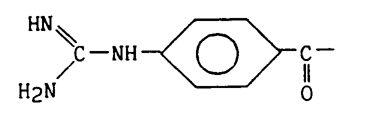 and
Arg represents the arginine residue,
Pro represents the proline residue,
Hyp represents the 4-hydroxyproline residue,
Gly represents the glycine residue,
Thia represents the β-(2-thienyl)alanine residue,
Ser represents the serine residue,
Tic represents the 1,2,3,4-tetrahydroisoguinoline-3-carbonyl residue, and
Oic represents the octahydroindole-2-carbonyl residue,
each amino acid in the peptide sequence having the D or L configuration at its α-carbon atom,
its enantiomers and diastereoisomers and also its addition salts with a pharmaceutically acceptable acid or base.

2. Compound of formula (I) according to claim 1 which is the compound of the formula:
G-Arg-Pro-Hyp-Gly-Thia-Ser-(D)Tic-Oic-Arg-OH
wherein:
(D)Tic represents the 1,2,3,4-tetrahydroisoquinoline-3-carbonyl radical having the D configuration at its α-carbon atom, the other amino acids in the peptide sequence having the L configuration at their α-carbon atom, Hyp has the 4R configuration, and Oic has the (2S,3aS,7aS) configuration,
and also its addition salts with a pharmaceutically acceptable acid or base.

3. Process for the preparation of the compound of formula (I) according to claim 1, characterised in that that compound can be obtained by solid-phase sequential synthesis starting from protected amino acids, by synthesis of peptide fragments in solution, or, where appropriate, by a combination of those two techniques.

4. Process for the preparation of the compound of formula (I) according to either claim 1 or claim 2, characterised in that that compound is obtained by solid-phase sequential synthesis to yield, starting from a resin substituted by Fmoc-Arg-OH, the unit H-Pro-Hyp-Gly-Thia-Ser-(D)Tic-Oic-Arg-resin, which is reacted directly with G-Arg(Pmc)-OH to yield, after deprotection, the compound of formula (I).

5. Pharmaceutical compositions comprising as active ingredient at least one compound of formula (I) according to either claim 1 or claim 2, on its own or in combination with one or more pharmaceutically acceptable excipients or carriers.

6. Pharmaceutical compositions according to claim 5 comprising at least one active ingredient according to either claim 1 or claim 2, for use as bradykinin antagonists in the treatment of traumas of various origins, inflammatory disorders, pain, shock, allergic disorders, bronchial hyperreactivity, and insufficient motility of sperm.

## Claims (Claims for the following Contracting State(s): ES)

1. Process for the preparation of the compound of formula (I):
G-Arg-Pro-Hyp-Gly-Thia-Ser-Tic-Oic-Arg-OH (I)
wherein:
G represents the 4-guanidinobenzoyl radical of the formula: 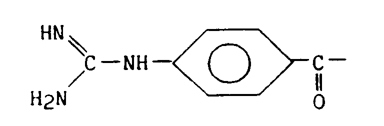 and
Arg represents the arginine residue,
Pro represents the proline residue,
Hyp represents the 4-hydroxyproline residue,
Gly represents the glycine residue,
Thia represents the β-(2-thienyl)alanine residue,
Ser represents the serine residue,
Tic represents the 1,2,3,4-tetrahydroisoquinoline-3-carbonyl residue; and
Oic represents the octahydroindole-2-carbonyl residue,
each amino acid in the peptide sequence having the D or L configuration at its α-carbon atom,
its enantiomers and diastereoisomers and also its addition salts with a pharmaceutically acceptable acid or base,
characterised in that that compound can be obtained by solid-phase sequential synthesis starting from protected amino acids, by synthesis of peptide fragments in solution, or, where appropriate, by a combination of those two techniques.

2. Process for the preparation of the compound of formula (I) according to claim 1 which is the compound of the formula:
G-Arg-Pro-Hyp-Gly-Thia-Ser-(D)Tic-Oic-Arg-OH
wherein:
(D)Tic represents the 1,2,3,4-tetrahydroisoquinoline-3-carbonyl radical having the D configuration at its α-carbon atom, the other amino acids in the peptide sequence having the L configuration at their α-carbon atom, Hyp has the 4R configuration, and Oic has the (2S,3aS,7aS) configuration,
and also its addition salts with a pharmaceutically acceptable acid or base.

3. Process for the preparation of the compound of formula (I) according to claim 1, characterised in that that compound can be obtained by solid-phase sequential synthesis to yield, starting from a resin substituted by Fmoc-Arg-OH, the unit H-Pro-Hyp-Gly-Thia-Ser-(D)Tic-Oic-Arg-resin, which is reacted directly with G-Arg(Pmc)-OH to yield, after deprotection, the compound of formula (I).

## Patentansprüche (Patentansprüche für folgende(n) Vertragsstaat(en): AT, BE, CH, DE, DK, FR, GB, GR, IE, IT, LI, LU, NL, PT, SE)

1. Verbindung der Formel (I):
G-Arg-Pro-Hyp-Gly-Thia-Ser-Tic-Oic-Arg-OH (I)
in der G die 4-Guanidinobenzoylgruppe der Formel: 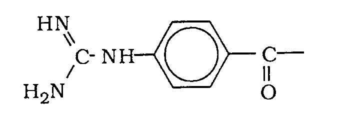 und Arg den Arginyl-Rest,
Pro den Prolyl-Rest,
Hyp den 4-Hydroxyprolyl-Rest,
Gly den Glycyl-Rest,
Thia den β-(2-Thienyl)-alanyl-Rest,
Ser den Seryl-Rest,
Tic den 1,2,3,4-Tetrahydroisochinolin-3-carbonyl-Rest und
Oic den Octahydroindol-2-carbonyl-Rest
bedeuten,
wobei jede Aminosäure der Peptidsequenz im Bereich ihres α-Kohlenstoffatoms die D- oder L-Konfiguration besitzt,
deren Enantiomere, Diastereoisomere sowie deren Additionssalze mit einer pharmazeutisch annehmbaren Säure oder Base.

2. Verbindung der Formel (I) nach Anspruch 1, nämlich die Verbindung der Formel:
G-Arg-Pro-Hyp-Gly-Thia-Ser-(D)Tic-Oic-Arg-OH
in der:
(D)Tic den 1,2,3,4-Tetrahydroisochinolin-3-carbonyl-Rest mit der D-Konfiguration im Bereich seines α-Kohlenstoffatoms darstellt und die anderen Aminosäuren der Peptidsequenz im Bereich ihres α-Kohlenstoffatoms die L-Konfiguration aufweisen. Hyp die Konfiguration 4R und Oic die Konfiguration (2S,3aS,7aS) besitzen,
sowie deren Additionssalze mit einer pharmazeutisch annehmbaren Säure oder Base.

3. Verfahren zur Herstellung der Verbindung der Formel (I) nach Anspruch 1, **dadurch gekennzeichnet**, daß sie durch sequentielle Synthese auf fester Phase ausgehend von geschützten Aminosäuren, durch Synthese von Peptidfragmenten in Losung oder gegebenenfalls durch eine Kombination dieser beiden Methoden erhältlich ist.

4. Verfahren zur Herstellung der Verbindung der Formel (I) nach einem der Ansprüche 1 oder 2, **dadurch gekennzeichnet**, daß sie durch sequentielle Synthese auf fester Phase erhalten wird, die ausgehend von einem mit Fmoc-Arg-OH substituierten Harz zu der Einheit H-Pro-Hyp-Gly-Thia-Ser-(D)Tic-Oic-Arg-Harz führt, das man direkt mit G-Arg(Pmc)-OH umsetzt, was nach der Abspaltung der Schutzgruppen zu der Verbindung der Formel (I) führt.

5. Pharmazeutische Zubereitungen enthaltend als Wirkstoff mindestens eine Verbindung der Formel (I) nach einem der Ansprüche 1 oder 2 allein oder in Kombination mit einem oder mehreren pharmazeutisch annehmbaren Trägermaterialien oder Bindemitteln.

6. Pharmazeutische Zubereitungen nach Anspruch 5 enthaltend mindestens einen Wirkstoff nach einem der Ansprüche 1 oder 2 geeignet als Bradykinin-Antagonisten bei der Behandlung von traumatischen Zuständen unterschiedlichen Ursprungs, Entzündungszuständen` Schmerzen, Schockzuständen, allergischen Störungen, Bronchienhyperreaktivität und Insuffizienzen der spermatozoiden Beweglichkeit.

## Patentansprüche (Patentansprüche für folgende(n) Vertragsstaat(en): ES)

1. Verfahren zur Herstellung der Verbindung der Formel (I):
G-Arg-Pro-Hyp-Gly-Thia-Ser-Tic-Oic-Arg-OH (I)
in der G die 4-Guanidinobenzoylgruppe der Formel: 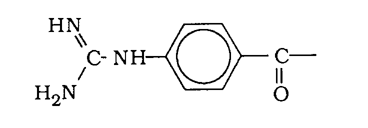 und Arg den Arginyl-Rest,
Pro den Prolyl-Rest,
Hyp den 4-Hydroxyprolyl-Rest,
Gly den Glycyl-Rest,
Thia den β-(2-Thienyl)-alanyl-Rest,
Ser den Seryl-Rest,
Tic den 1,2,3,4-Tetrahydroisochinolin-3-carbonyl-Rest und
Oic den Octahydroindol-2-carbonyl-Rest
bedeuten,
wobei jede Aminosäure der Peptidsequenz im Bereich ihres α-Kohlenstoffatoms die D- oder L-Konfiguration besitzt,
von deren Enantiomeren, Diastereoisomeren sowie deren Additionssalzen mit einer pharmazeutisch annehmbaren Säure oder Base,
**dadurch gekennzeichnet**, daß sie durch sequentielle Synthese an fester Phase ausgehend von geschützten Aminosäuren, durch Synthese von Peptidfragmenten in Lösung oder gegebenenfalls durch eine Kombination dieser beiden Methoden erhältlich ist.

2. Verfahren zur Herstellung der Verbindung der Formel (I) nach Anspruch 1, nämlich der Verbindung der Formel:
G-Arg-Pro-Hyp-Gly-Thia-Ser-(D)Tic-Oic-Arg-OH
in der:
(D)Tic den 1,2,3,4-Tetrahydroisochinolin-3-carbonyl-Rest mit der D-Konfiguration im Bereich seines α-Kohlenstoffatoms darstellt und die anderen Aminosäuren der Peptidsequenz im Bereich ihres α-Kohlenstoffatoms die L-Konfiguration aufweisen, Hyp die Konfiguration 4R und Oic die Konfiguration (2S,3aS,7aS) besitzen,
und von deren Additionssalzen mit einer pharmazeutisch annehmbaren Säure oder Base.

3. Verfahren zur Herstellung der Verbindung der Formel (I) nach Anspruch 1, **dadurch gekennzeichnet**, daß sie erhältlich ist durch sequentielle Synthese an fester Phase, die ausgehend von einem durch Fmoc-Arg-OH substituierten Harz zu der Einheit H-Pro-Hyp-Gly-Thia-Ser-(D)Tic-Oic-Arg-Harz führt, das man direkt mit G-Arg(Pmc)-OH umsetzt, was nach der Abspaltung der Schutzgruppenzu der Verbindung der Formel (I) führt.
